# EUROPEAN PATENT APPLICATION

(11) **EP 1 447 094 A1**
(43) Date of publication of application: **18.08.2004**
(21) Application number: 02777899.2
(22) Date of filing: 22.10.2002
(51) Int. Cl.: A61K 45/00, A61K 31/164, A61K 31/405, A61K 9/06, A61K 9/08, A61P 17/04, A61P 27/02, A61P 27/14, A61P 31/00

(54) **REMEDIES FOR PRURITUS**

(30) Priority: 22.10.2001 JP 2001323218
(71) Applicant: SANTEN PHARMACEUTICAL CO., LTD., Higashiyodogawa-ku, Osaka-shi, Osaka 533-8651 (JP)
(72) Inventor: MIYAJI, Suguru, c/o SANTEN PHARMACEUTICAL CO. LTD., Ikoma-shi, Nara 630-0101 (JP); KATO, M., c/o SANTEN PHARMACEUTICAL CO., LTD., Ikoma-shi, Nara 630-0101 (JP)
(74) Representative: Merkle, Gebhard
(86) International application number: PCT/JP2002/010912
(87) International publication number: WO 2003/035109

(57) **Abstract**

The invention relates to chewing gum or soft caramel and the production method thereof. The inventive method consists in producing a continuous strand of a product comprising chewing gum or soft caramel, said strand being in the form of a channel. A sheet comprising a liquid product that gels in a reduced amount of time is deposited inside the channel. Said method can be used to produce individual units of said product by cutting the aforementioned compound strand. The sheet of gelled product is perfecly visible in each of said units as it remains on the surface of three of the sides of the chewing gum, said gum being laterally fixed to the sheet of gel in order to prevent the sheet from separating easily.

## Description

### Field of the Invention

This invention refers to a confectionery product and a method for obtaining it. More concretely, the invention refers to a chewing gum or soft candy on which a visible gel is superficially stuck and its production method in a non-stop process.

### Background of the invention

Confectionery products are known composed of two differentiated pieces: an external and an internal parts. The external part can be composed of chewing gum, soft candy or hard candy or other confectionery product which can be extruded. The internal part can also be made of chewing gum, gel, soft candy or hard candy or an effervescent product, in solid, liquid pasty or pulverulent state, according to each case.

Generally, the end product is obtained by dividing into unitary pieces a continuous strand formed from an external tube and by simultaneously introducing in it the centre product.

For such aim, methods are used which are partly disclosed in US patent 3,857,963 consisting in extruding the external product, in this case chewing gum and at same time the centre product, in this case a liquid, both initially located in two separate containers. The extrusion of the chewing gum is carried out through a nozzle which concentrically integrates a second nozzle though which the centre material is located in the chewing gum tube. Other patents referring to similar proposals are US 4,466,983 and US 5,035,905.

Spanish patent n° 92011551, discloses a chewing gum filled with gel and the method for obtaining it. In that patent the gel is as a centre product and is only visible from the ends of each piece subsequently to cutting the continuous strand into individual units.

The unitary pieces of such chewing gums have the feature that the centres remain occluded because the mass of the material contained within them will be liquid or powdered or at most it can be seen at the ends of each piece subsequently to cross cutting a continuous strand into individual pieces.

Unlike the centres for hard candies, the centres for chewing gums must have a softness similar to that of the chewing gum itself because consuming it necessarily carries to be able to chew it and homogenize both pieces. Industrial obtention thereof is significantly different from that applied for hard candies due to the significant softness of the chewing gum itself versus the little softness of the latter ones.

Usually, pulverulent or liquid state centres are used for chewing gums. In both cases, it is not possible to apply a non-stop production but units have to be individually wrapped, which means longer processes than for producing chewing gum without centre.

Also chewing gums or other sweets can be found formed by two superposed layers of different colour and/or taste, which basically are the same kind of product (chewing gum, gel or chewable soft candy). Mounting or superposing the layers is usually manual or through double extrusion.

For obtaining gelled products it is necessary to individually deposit a product in liquid state in moulds, having the product resting a more or long period of time within chambers having special air conditioning for gelling and drying it.

Thereafter, it is necessary to produce each of the final pieces, fully coating them with the chewing gum itself.

Preparing separately the mentioned gelled centre prevents to produce chewing gums in a non stop process with that kind of material for centre or for coating the surface.

In concrete combinations of solid coating and solid centre, it is possible that the end product can show a configuration allowing that its centre can be seen from outside. Chewing gums are known formed of two compounds of same material, obtained from concentrically extruding two different masses of such product, such as those disclosed in the British Patent 2096447 B. Soft candies having analogous characteristics are also known. However, in all of them, the centre can only be seen at the ends where the pieces have been cut.

However, no chewing gum or soft candy is known having a surface layer of a gel mass stuck to the chewing gum itself, so that such gel layer is totally visible on the face of each piece. Obviously, processes for non stop production of soft candies or chewing gums are known either having such characteristics.

### Description of the invention

This invention refers to a chewing gum or soft candy having a layer of gel on one of its faces, fully visible from outside and to the method for producing it in a non stop process. The chewing gum or soft candy is obtained by cutting into pieces a strand composed of a chewing gum or soft candy support which surface includes the gel deposited on it.

The unitary end product has a sufficient and stable consistence and it can integrate different shapes, with different compositions of tastes and different colours.

The superficial layer is constituted of a liquid product which is gelled within a short period of time. This product is constituted of sugars, aromas, glucose syrup and other ingredients, including one or several gelling agents such as starches, modified starches, agar-agar, pectins having a high and low level of methoxyl, carrageenates, gelatins and the like.

The method used, as it will be described, allows to obtain individual units by forming a continuous channel of extruded chewing gum or soft candy, which on its surface comprises a gel. Such method comprises different steps: separately preparing the three constituents (mass of chewing gum or soft candy, a liquid solution ranging from 93% to 99.5% and an acid solution ranging from 0.5% to 7%, extruding the chewing gum or soft candy forming a channel, pumping the liquid solution and pumping the acid solution and through a mixer, depositing it in liquid state on the surface of such channel, gelling the centre and non stop processing.

The own mass of the chewing gum or soft candy is conventional, prepared according to well-known processes and unconnected with this invention.

The gel product is simultaneously prepared and in liquid state by mixing a liquid solution with an acid solution. Gelling the centre product introduced in liquid state occurs in a short interval of time, thanks to adding acid and/or cooling thereafter.

At same time the chewing gum or soft candy mass which forms the channel is extruded its surface also receive by pumping the liquid solution and the acid solution through a static mixer forming this way the gel layer, in liquid form, which remains trapped on the surface of the chewing gum or soft candy channel.

To facilitate the gel layer is deposited and stuck to the surface of the chewing gum or chewable soft candy strand, it is essential that such strand has a U-shaped section and that the sides of such strand are fully closed on the sides of the gel layer. This prevents that in further product handling and cooling, the gel layer contacts the machines and therefore is stuck to them.

More concretely, the chewing gum or soft candy extruding nozzle has a given shape for the exit of the product which makes that the chewing gum or soft candy channel has a dovetail-shaped cross section and its height is higher than that of the surface of the gel layer. This way it is achieved that the gel layer can be trapped against the chewing gum or soft candy channel and, in addition it does not contact and therefore does not stick to the different components of the production line through which the continuous strand has to pass until it is cut into individual pieces and wrapped.

The mixture of liquid solution and acid solution (which as a whole forms a liquid mass which will provide the gel) is pumped at a temperature higher than the gelling temperature, the deposited liquid acquiring a definitive consistence as a gel on the surface of the said channel-shaped strand, within a short interval of time, by reaction of the liquid solution with the acid solution and/or by decreasing the temperature of the whole.

This way, a continuous strand of product composed of chewing gum or soft candy and a surface gel is obtained. Such strand, when cooled (as usual in chewing gum production) can be divided into individual portions. On each of such portions, the gel layer will be fully visible on three of the faces of the product.

### Preferred embodiments of the invention

Concrete embodiments of the object of this invention will make apparent the characteristics thereof as well as the advantages it can provide.

The examples described below are based on a mass of chewing gum or soft candy which on its surface can comprise a layer made of a liquid solution and an acid solution.

A mass of chewing gum can be constituted of:
50 to 75% crushed sugar
15 to 30% basic gum
10 to 20% glucose syrup
0.2 to 2% aromas, and
1 to 8% glycerine.

Such chewing gum mass can also comprise up to 3% of citric acid and up to 1% of dyes.

It is prepared as follows. A part of crushed sugar, the basic gum and the glucose syrup are mixed at a temperature of 60 centigrade degrees, adding thereafter the dye, if any. Then the glycerine and, when applicable, the citric acid, the rest of the crushed sugar and last the aromas are added. This mass will be ready to be extruded.

A mass of soft candy is constituted of:
20 to 40% sugar
20 to 40 % glucose syrup
6 to 9% water
6 to 10% fat
0.1 to 2% emulsifier
0.5 to 3% gelatin
0.01 to 2% aromas

This mass of soft candy can also comprise up to 5% of citric acid and up to 1% of dye.

It is prepared as follows. 5% of glucose syrup is mixed with 2% of water at a temperature ranging from 30 to 35 centigrade degrees; then the gelatin is added until dissolution is achieved, increasing its temperature up to 50-60 centigrade degrees.

Separately, the sugar is mixed with the rest of the glucose syrup and the rest of water at a temperature ranging from 120 to 135 centigrade degrees. When it is obtained, it is cooled up to 90 centigrade degrees.

The former mixture is added to this later together with the fat and the emulsifier and when it has been mixed, the preparation is cooled to 60 centigrade degrees, the aromas are added to it, they are mixed and it is left to rest about eight hours at a temperature ranging from 25 to 30 centigrade degrees.

A liquid solution is constituted of:
20 to 60% sugar
20 to 50% glucose syrup
0 to 15% glycerine
0.5 to 5% HM pectin
0. 1 to 5% sodium citrate
0.1 to 5% citric acid
0.01 to 2% aromas

This solution can also comprise up to 1% of dyes.

It is prepared by dissolving the pectin, the sodium citrate and the citric acid in water at a temperature ranging from 50 to 80 centigrade grades, without the pectin become gelled nor degraded. Sugar, glucose syrup and the former preparation are separately mixed, they are boiled to yield a dry matter ranging from 75% to 82% in order that a proportion of water ranging from 18% to 25% is left. Then glycerine, aroma and dye, if any, are added.

An acid solution is constituted of:
40 to 90% water
10 to 60% acid

This solution can also comprise up to 2% of dye.

It is prepared by mixing its compounds at a temperature ranging from 15 to 25 centigrade degrees.

The mass of chewing gum or soft candy is prepared as stated and it is placed in a container having an outlet, it can be mechanically pumped through an extrusion press having at its end an outlet ending in a nozzle which can continuously produce a channel-shaped strand having a dovetail-shaped cross section to facilitate that the side walls of such channel trap or seize thereafter the gel.

The liquid solution and the acid solution are placed in two separate containers, provided with an outlet duct with an individual pump on each. The two pumps are connected to a static mixer.

The static mixer is provided with an outlet having a duct (of a smaller section than that of the nozzle of the extrusion press for the mass of the chewing gum or soft candy) such outlet being flat and it is located on the surface of the valley of the U-shaped nozzle of the extrusion press.

Unavoidably, the chewing gum mass strand has to be channel-shaped to receive within it the liquid mass which will be gelled thereafter.

When extruding the chewing gum mass, the gellable centre will be simultaneously deposited which will spread on almost the full valley of the chewing gum or chewable soft candy strand.

By including the organic acid in the composition of the centre product will provoke that the pectin with a high index of methoxyl is gelled within a short interval of time. Such period of time will mainly depend on the amount of organic acid added, on the concentration of pectin stated and on the general temperature of the solution during and after the addition of the organic acid. More concretely, the gelling time is significantly decreased when the temperature of the static mixer outlet decreases, so that by adjusting the amount of the organic acid added, the length of the outlet duct or the extrusion temperature of the mass of the chewing gum or soft candy, among other factors, it is possible to achieve that the solution is gelled on the surface of the mass of the chewing gum or soft candy.

A similar process can be used in the event of using a pectin having a low index of methoxyl. In this case, gelling is obtained through a calcium salt aqueous solution having a high concentration of diatomic ions Ca++.

A second embodiment for obtaining a preparation suitable for its use as a surface layer and having analogous characteristics to those described, can consist in a solution of sugar and syrup and aromas with a gelling agent such as agar-agar, carrageenates, starches or gelatins, or mixtures therefrom.

This solution is kept hot over the gelling temperature in the static mixer, until the moment it is deposited simultaneously with the extrusion of the chewing gum or soft candy strand.

A decrease of the temperature in the outlet duct of the mixer allows to cool the solution to achieve it is already gelled on the surface of the chewing gum mass strand.

Cooling the strand when the whole of it has been produced allows to process the whole of it.

The percentages of the chewing gum or soft candy mass and of the gellable layer with respect to the end product are variable, and the bottom of the strand and its side walls having to be sufficiently strong to sustain the gellable layer preventing it is scattered before it is gelled. The minimum percentage of chewing gum or soft candy mass must be at least 40% the total product constituted by the chewing gum or soft candy and the gellable mass.

Regardless the kind of gellable mass used, the result is a channel of chewing gum or soft candy filled with a gelled product and the whole of which can be cut into individual pieces showing on one of the larger faces of each piece the gelled layer and the superficial part of the walls of the channel of chewing gum or soft candy. On the opposite face and on the side faces only chewing gum or chewable soft candy can be seen. And last, on both faces of the cut of the individual pieces chewing gum or chewable soft candy and gelled layer can be seen.

The resulting confectionery product (chewing gum or soft candy with gel) and the method for obtaining it, in its essential part could be modified in its incidental characteristics, by a man of the art without modifying the spirit of the invention which is detailed in following claims.

## Claims

1. A confectionary product **characterized in that** it takes the shape of a channel filled with a gel such channel being formed of chewing gum or soft candy and the gel contained in such channel is visible from outside on three faces of each piece.

2. A confectionary product according to above claim, **characterized in that** it is obtained from cutting into pieces a channel-shaped continuous strand of chewing gum or soft candy filled with a gel.

3. A confectionary product according to above claims, **characterized in that** the gel is visible on three sides of the piece formed of chewing gum or soft candy, two of them being opposite.

4. A confectionary product according to above claims **characterized in that** the gel is located on the surface of the mass of chewing gum or soft candy

5. A confectionary product according to above claims, **characterized in that** the gel represents up to 60% the total volume of the end product.

6. A confectionary product according to claim 2, **characterized in that** such continuous strand has a U-shaped cross section and the sides of the strands are closed on the sides of the layer of gel.

7. A confectionary product according to claim 6, **characterized in that** such continuous strand has a dovetail-shaped cross section.

8. A confectionary product according to claim 2, **characterized in that** the piece of chewing gum or soft candy and the gel have regular geometrical shapes.

9. A confectionary product according to above claims, **characterized in that** the gel visible from outside has one colour and taste or more than one colour and/or more than one taste.

10. A confectionary product according to above claims, **characterized in that** the chewing gum or the chewable soft candy has one colour and taste or more than one colour and/or more than one taste.

11. Method for obtaining a confectionary product **characterized in that** it comprises following and successive steps:
a. preparing separately a mass of chewing gum or soft candy, a liquid solution and an acid solution
b. placing the three elements in separate containers
c. pumping separately the liquid solution and the acid solution to a static mixer
d. extruding the mass of chewing gum or soft candy though a nozzle forming a continuous strand having a U-shaped cross section
e. including inside the whole channel the mixture of the liquid solution and the acid solution in solid state; and
f. cooling the continuous strand composed of a channel-shaped mass of chewing gum or soft candy and a mass of superficial layer-shaped gel until this later is gelled.

12. Method for obtaining a confectionary product according to claim 11, **characterized in that** the mass of gel is gelled within the chewing gum or soft candy channel.

13. Method for obtaining a confectionary product according to claim 11, **characterized in that** such continuous strand possesses a dovetail-shaped cross section.

14. Method for obtaining a confectionary product according to claim 11, **characterized in that** the gelling time is shorter than the time necessary for accurately cooling the chewing gum or soft candy strand for processing such strand.

15. Method for obtaining a confectionary product having the shape of a channel filled with gel, according to above claims, **characterized in that** the strand obtained can be formed as individual pieces, by cutting the strand into portions.
